# EUROPÄISCHE PATENTANMELDUNG

(11) **EP 0 962 234 A1**
(43) Veröffentlichungstag der Anmeldung: **08.12.1999**
(21) Anmeldenummer: 98120812.7
(22) Anmeldetag: 03.11.1998
(51) Int. Cl.: A61N 1/36, A61N 1/34

(54) **Reizstromgerät zur transkutanen elektrischen Stimulation von Nerven und Muskeln**

(30) Priorität: 05.06.1998 DE 29810076 U
(71) Anmelder: Pierenkemper GmbH, 35630 Ehringshausen (DE)
(72) Erfinder: Kreutner, Bernd Jürgen, 35630 Ehringshausen-Daubhausen (DE)
(74) Vertreter: Linser, Heinz

(57) **Zusammenfassung**

Die Erfindung betrifft ein Reizstromgerät zur transkutanen elektrischen Stimulation von Nerven und Muskeln zur Therapie, bestehend aus herkömmlichen aktiven elektronischen Bauelementen, sowie einem Generator mit nachgeschalteter Endstufe und einem als Transformator ausgebildeten Impulsübertrager, welcher nach einer steilen Anstiegsflanke eine weitgehend konstante elektrische Ladung bei möglichst konstanter Amplitude abgibt und nach Beendigung der Reizzeit die abgegebene Ladung wieder eliminiert. Zu dem Impulsübertrager (3) sind eine Speichervorrichtung (4) und ein Schalter (5) mit Ausgangsbuchsen (6) in Reihe geschaltet, wobei eine mit dem Generator (1) verbundene Steuerung (7) den Schalter (5) zeitgerecht mit einer Zusatzenergie beaufschlagt, derart, daß der Anstiegsamplitude der Ausgangsimpulse zur Steigerung ihrer Anstiegsgeschwindigkeit eine höherfrequente Spannung überlagerbar ist. Der Schalter (5) mit der erforderlichen Schaltgeschwindigkeit und Spannungsfestigkeit ist zwischen der Sekundärseite des Impulsübertragers (3) und den Ausgangsbuchsen (6) angeordnet. Auf der Sekundärseite des Impulsübertragers (3) ist zur Zwischenspeicherung der Energie des Ausgangsimpulses ein Speicherbauteil (4) zwischengeschaltet.

## Beschreibung

Die Erfindung betrifft ein Reizstromgerät zur transkutanen elektrischen Stimulation von Nerven und Muskeln zur Therapie, bestehend aus herkömmlichen aktiven elektronischen Bauelementen, sowie einem Generator mit nachgeschalteter Endstufe und einem als Transformator ausgebildeten Impulsübertrager, welches nach einer steilen Anstiegsflanke eine weitgehend konstante elektrische Ladung bei möglichst konstanter Amplitude abgibt und nach Beendigung der Reizzeit die abgegebene Ladung wieder eliminiert.

Reizgeräte der oben beschriebenen Art sind unter der Bezeichnung TENS-Geräte bekannt, beispielsweise aus der DE-3734036 A1.

TENS-Geräte stimulieren mit Hilfe von elektrischer Energie die elektrischen Reizleiter des menschlichen Körpers, nämlich die Nerven und die Muskeln. Dabei ist es unerheblich, ob diese Energie von einer Strom- oder einer Spannungsquelle geliefert wird.

Die bekannten TENS-Geräte erzeugen näherungsweise Rechteckimpulse mit einer Impulsdauer von ca. 200 µs, die in ihrer Intensität einstellbar sind. Die Frequenz der Rechteckimpulse liegt im Bereich <1 Hz ... 120 Hz. Bei den auf dem Markt befindlichen Geräten handelt es sich größtenteils um tragbare, batteriegespeiste Apparate, die maximale Impulsströme von ca. 50 mA applizieren.

Bei der Entwicklung der TENS-Geräte fand die Auswirkung der Körperimpedanz des Menschen auf die Kurvenform der abgegebenen Impulse und damit auf die Wirksamkeit der Therapie bisher nur wenig Beachtung.

In der Literatur und in den Gebrauchsanweisungen von TENS-Geräten findet man häufig die idealisierte Kurvenform eines Rechtecks. Ein idealer Rechteckimpuls zeichnet sich durch unendlich kurze Anstiegs- und Abfallzeiten, einen gleichbleibenden, maximal gewählten Intensitätswert während der Impulsdauer und eine gleichbleibende Impulsform an allen Lastwiderständen aus. Eine derartige ideale Impulsform wäre jedoch nur mit idealen Bauteilen zu erzielen, die es jedoch in der Realität nicht gibt. Man kann nur versuchen, sich so gut wie möglich den Anforderungen an die ideale Kurvenform anzunähern.

Die Ausgangssignale eines TENS-Gerätes sind wesentlich abhängig von der Lastimpedanz, d.h. von der Art des Widerstandes, der an das TENS-Gerat angeschlossen ist. Der menschliche Körper stellt, als Lastwiderstand eines TENS-Gerätes, einen komplexen Widerstand der, der in einem Ersatzschaltbild zusammengefaßt werden kann. Komplexe Widerstände sind Widerstände, deren Wert zeitabhängig ist und die zusätzlich einen reellen Anteil besitzen. Komplexe Widerstände können in sogenannten Ersatzschaltbilder von ohmschen, reellen Widerständen sowie Induktivitäten und Kapazitäten beschrieben bzw. dargestellt werden. Diese Kombinationen werden allgemein als Impedanzen bezeichnet. Streng genommen stellen alle realen Bauteile komplexe Widerstände dar, es ist nur eine Frage der Größenordnung der einzelnen Komponenten.

Reelle Widerstände, auch ohmsche Widerstände genannt, stellen eine Sonderform der komplexen Widerstände dar. Der Einfluß ihrer Induktivitäten z.B. der Anschlußdrähte und der Kapazitäten, wie z.B. die Strecke zwischen den Anschlüssan, ist im betrachteten Frequenzbereich vernachlässigbar.

Um TENS-Geräte mit hoher Therapiewirkung zu entwickeln, genügt es daher nicht, daß sie an einem reellen Widerstand eine möglichst gute Annäherung an die ideale Impulstorm erzielen. Vielmehr ist bei der Entwicklung eines Gerätes der komplexe Widerstand des Patientenkörpers zu beachten, um ihn mit einem maximalen Wirkungsgrad therapieren zu können.

Da der Körper individuell sehr verschiedene Werte besitzt, ist nur näherungsweise die Darstellung eines Ersatzschaltbildes möglich, das allgemeine Gültigkeit hat. In den USA wurde ein Ersatzschaltbild entwickelt, das die Meßergebnisse an einer Vielzahl von Personen zusammenfaßt. Es dient zu Messungen gemäß der amerikanischen Norm AAMI an transkutanen elektrischen Nervenstimulatoren. Die Brauchbarkeit dieses Ersatzschaltbildes konnte eigenen Messungen nach bestätigt werden. Es zeigt neben reellen Komponenten einen eindeutig kapazitiven Charakter.

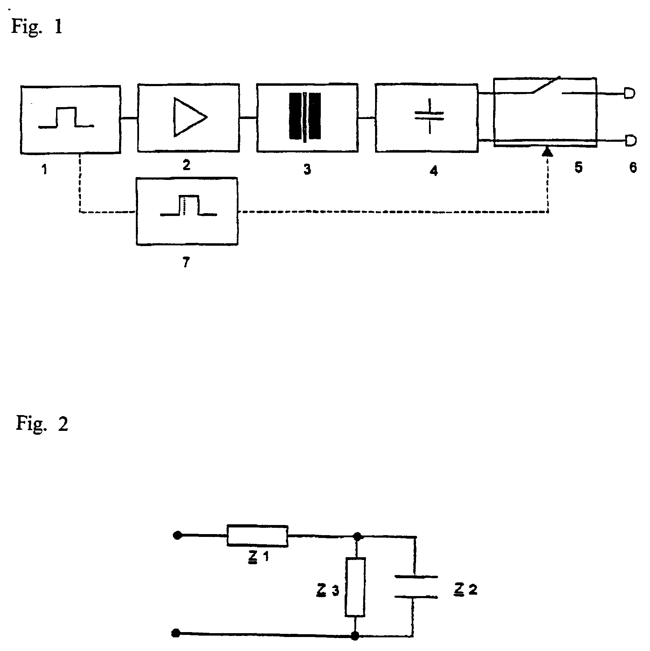

Das Ersatzschaltbild des Körpers nach ANSI/AAMI, welches in Figur 2 dargestellt ist, besteht aus einem Widerstand Z1 mit einer in Reihe liegenden Kapazität Z2, zu der ein Widerstand Z3 parallel geschaltet ist.

Dies ist ein relativ einfach gehaltenes Ersatzschaltbild, die Wirkung auf das Ausgangssignal wird aber relativ gut wiedergegeben.

Nach Figur 2 bedeuten:
- -Z1: den Übergangswiderstand zwischen Elektroden und Haut sowie zwischen Ober- und Unterhaut. Der Wert schwankt individuell von 200 bis 1 000 Ohm;
- -Z2: den kapazitiven Anteil des Körpers. Er wird durch verschiedene, gut leitende Haut- und Gewebeschichten gebildet, getrennt durch schlecht leitende Schichten. Dadurch werden in den gut leitenden Schichten Ladungen gespeichert. Die mittlere Kapazität beträgt 100 nF.
- -Z3: den Übergangswiderstand zwischen den gut leitenden Gewebeschichten. Er sorgt für die Entladung der gut leitenden Schichten und liegt relativ fest bei 2,7 kOhm.

Zu bachten ist, daß dies lediglich ein Ersatzschaltbild für Impulsformen ist und keine Gültigkeit für Gleichströme bzw. - spannungen hat. Es ist daher nicht möglich, dieses Ersatzschaltbild mit einem gewöhnlichen Ohmmeter zu messen.

Der Erfindung liegt nun die Aufgabe zugrunde die Wirksamkeit der Reizstromgeräte für Therapiezwecke zu erhöhen, indem die Anstiegsgeschwindigkeit der Impulse, mit denen der menschlichen Körper beaufschlagt wird, erhöht wird.

Die Lösung dieser Aufgabe besteht gemäß der Erfindung darin, daß bei dem eingangs aufgeführten Reizstromgerät dem Impulsübertrager eine Speichervorrichtung und ein Schalter mit Ausgangsbuchsen in Reihe zugeordnet ist, wobei eine mit dem Generator verbundene Steuerung den Schalter zeitgerecht mit einer Zusatzenergie beaufschlagt, derart, daß der Anstiegsamplitude der Ausgangsimpulse zur Steigerung ihrer Anstiegsgeschwindigkeit eine höherfrequente Spannung überlagerbar ist.

In Weiterbildung der Erfindung ist der genannte Schalter mit der erforderlichen Schaltgeschwindigkeit und Spannungsfestigkeit zwischen der Sekundärseite des Übertragers und den Ausgangsbuchsen angeordnet.

Auf der Sekundärseite des Impulsübertragers ist zur Zwischenspeicherung der Energie des Ausgangsimpulses nach der Erfindung ein Speicherbauteil zwischengeschaltet.

Die Erfindung wird anhand der Zeichnung näher erläutert. Hierbei zeigen:
- Figur 1: ein Blockschaltbild eines TNS-Gerätes in schematischer Darstellung mit einer Einschaltflankenkorrekturschaltung (EKS) nach der Erfindung und
- Figur 2: ein Ersatzschaltbild der Impedanz des menschlichen Körpers.

Das Reizstromgerät nach der Erfindung wird mit seiner Einschaltflankenkorrekturschaltung (EKS) anhand eines Blockschaltbildes in schematischer Darstellung erläutert. Hierbei bedeuten 1 die Energiequelle bzw. den Generator, 2 die Endstufe, 3 den Impulsübertrager bzw. Transformator, 4 eine Speichervorrichtung, 5 einen Schalter, 6 Ausgangsbuchsen und 7 die Steuerung für den Schalter 5.

Reizstromgeräte zur transkutanen elektrischen Stimulation von Nerven und Muskeln bringen elektrische Ladungen in Form von Stromimpulsen in den menschlichen Körper ein. Hierzu wird, bei batteriebetriebenen Geräten zur Heimtherapie, die notwendige Spannung mittels Impulsübertrager 3 erzeugt.

Das elektrische Ersatzschaltbild des menschlichen Körpers für Impulsströme, wie in Figur 2 wiedergegeben, stellt eine komplexe Impedanz aus reellen Wirkwiderständen und kapazitivem Anteil der. Die Körperimpedanz hat somit einen kapazitiven Charakter, also eine Speicherwirkung. Dementsprechend wird das Einbringen einer bestimmten elektrischen Ladungsmenge in den menschlichen Körper eine bestimmte Zeit dauern, abhängig von der Ausgangscharakteristik des TENS-Gerätes.

Die in eine Kapazität eingebrachte Ladungsmenge ist generell direkt der an dieser Kapazität zu messenden Spannung proportional. Die an den Patientenkörper angelegte Spannung wird bei den meisten handelsüblichen TENS-Geräten nach ca. 200 µs in Abhängigkeit von den entsprechenden Übergangswiderständen erreicht. Zum Vergleich sei erwähnt, daß die angelegte Spannung an einem rein reellen Wirkwiderstand als Last bereits nach ca. 10 µs erreicht wird. Die Grundvoraussetzung dafür ist, daß das TENS-Gerät überhaupt in der Lage ist, schnellere Anstiegezeiten zu erzeugen.

Ein Bauteil des TENS-Gerätes, das die Geschwindigkeit des Spannungsanstiegs prägend begrenzt, ist der verwendete und als Transformator ausgebildete Impulsübertrager, der aber in der Schaltung dazu benötigt wird, um zum einen die erforderliche galvanische Trennung zur Primärseite zu bewirken und zum anderen die erforderliche höhere Ausgangsspannung zu erzeugen. Da also auf dieses Bauteil nicht verzichtet werden kann, andererseits aber dieses Bauteil die Hauptursache für die begrenzte Anstiegsgeschwindigkeit der Ausgangsimpulse ist, muß gemäß der Erfindung die Einschaltflanke der Ausgangsimpulse korrigiert werden.

Hierzu wird nach der Erfindung in die Verbindung zwischen der Sekundärseite des Impulsübertragers 3 und der Ausgangsbuchse 6 ein schalter 5 eingefügt, der die erforderliche Schaltgeschwindigkeit und Spannungsfestigkeit aufweist. Die benötigte Energie des Ausgangsimpulses auf der Sekundärseite des Impulsübertragers wird zunächst in einem Energiespeicher 4 auf der Sekundärseite zwischengespeichert. Zu einem festgelegten Zeitpunkt wird der Schalter 5 geschlossen und der Ausgang aus dem Energiespeicher 4 und zusätzlich aus dem ImpulsÜbertrager 3 gespeist.

Die EKS nach der Erfindung bewirkt somit einen wesentlich schnelleren Anstieg sowohl an reeller Last als auch an einem komplexen Lastwiderstand. Hier sind Anstiegsgeschwindigkeiten von < 1 µs am Patienten möglich. Die angelegte Spannung wird also wesentlich früher erreicht, wodurch die Leistung des Gerätes und damit die Therapiewirkung erheblich gesteigert wird.

## Patentansprüche

1. Reizstromgerät zur transkutanen elektrischen Stimulation von Nerven und Muskeln zur Therapie, bestehend aus herkömmlichen aktiven elektronischen Bauelementen, sowie einem Generator mit nachgeschalteter Endstufe und einem als Transformator ausgebildeten Impulsübertrager, welches nach einer steilen Anstiegsflanke eine weitgehend konstante elektrische Ladung bei möglichst konstanter Amplitude abgibt und nach Beendigung der Reizzeit die abgegebene Ladung wieder eliminiert, **dadurch gekennzeichnet, daß** dem Impulsübertrager (3) eine Speichervorrichtung (4) und ein Schalter (5) mit Ausgangsbuchsen (6) in Reihe geschaltet sind, wobei eine mit dem Generator (1) verbundene Steuerung (7) den Schalter (5) zeitgerecht mit einer Zusatzenergie beaufschlagt, derart, daß der Anstiegsamplitude der Ausgangsimpulse zur Steigerung ihrer Anstiegsgeschwindigkeit eine höherfrequente Spannung überlagerbar ist.

2. Reizstromgerät nach Anspruch 1, **dadurch gekennzeichnet, daß** der Schalter (5) mit der erforderlichen Schaltgeschwindigkeit und Spannungsfestigkeit zwischen der Sekundärseite des Impulsübertragers (3) und den Ausgangsbuchsen (6) angeordnet ist.

3. Reizstromgerät nach Anspruch 2, **dadurch gekennzeichnet, daß** auf der Sekundärseite des Impulsübertragers (3) zur Zwischenspeicherung der Energie des Ausgangsimpulses ein Speicherbauteil (4) zwischengeschaltet ist.
